# EUROPEAN PATENT SPECIFICATION

(11) **EP 4 217 336 B1**
(45) Date of publication and mention of the grant of the patent: **17.12.2025**
(21) Application number: 21778238.2
(22) Date of filing: 21.09.2021
(51) Int. Cl.: C07C 51/09, C07C 69/716, C07C 59/185, C07D 307/44, C07D 307/48, C07C 51/00, C07C 67/00

(54) **PROCESS FOR PRODUCING LEVULINIC ACID**
VERFAHREN ZUR HERSTELLUNG VON LEVULINSÄURE
PROCÉDÉ DE PRODUCTION D'ACIDE LÉVULINIQUE

(30) Priority: 23.09.2020 IT 202000022387
(43) Date of publication of application: 02.08.2023
(73) Proprietor: BIO ON S.p.A., 10128 Torino (IT)
(72) Inventor: BEGOTTI, Simone, 46029 Suzzara (MN) (IT); MASSI, Alessandro, 40033 Casalecchio di Reno (BO) (IT); RAGNO, Daniele, 40127 Bologna (IT); DI CARMINE, Graziano, 44121 Ferrara (IT); LEONARDI, Costanza, 47521 Cesena (FC) (IT); BORTOLINI, Olga, 33010 Tavagnacco (UD) (IT)
(74) Representative: Bottero, Carlo
(86) International application number: PCT/IB2021/058572
(87) International publication number: WO 2022/064351

(56) References cited:
- WO-A1-2012/162001
- US-A1- 2012 302 767

## Description

The present invention is defined in the claims and relates to a process for producing levulinic acid starting from arabinose or xylose.

In recent years, the need has been felt to find new "platform molecules" that can be obtained through production processes that have a lower environmental impact than processes that involve the use of substances of fossil origin. These platform molecules are compounds suitable for the production of other molecules for use in various fields of chemistry.

A molecule useful for this purpose is levulinic acid, which can be used to obtain derivatives that have application in the chemical industry in a broad sense, or, more specifically, in the pharmaceutical, agrochemical and cosmetic sectors, and in fertilizers, plastics and polymers.

Levulinic acid (also known as 4-oxopentanoic acid or γ-ketovaleric acid) is an organic product with the formula HO-CO-(CH₂)₂-CO-CH₃ that can be widely used in chemical industry, in particular as an intermediate for the production of a wide variety of products such as resins, plasticizers, herbicides, solvents, fuel additives, flavorings, pharmaceuticals, and others.

Currently the most frequently used process for the production of levulinic acid involves the treatment of carbohydrates at acidic pH and at high temperature (up to 200°C), using a strong acid such as hydrochloric acid or sulfuric acid as a catalyst. For example, starting from sucrose, the most used reaction scheme that leads to formation of levulinic acid is the following:
- sucrose (C₁₂H₂₂O₁₁) + H₂O → glucose (C₆H₁₂O₆) + fructose (C₆H₁₂O₆);
- fructose (C₆H₁₂O₆) → HMF (C₆H₆O₃) + 3 H₂O;
- HMF (C₆H₆O₃) + 2 H₂O → levulinic acid (C₅H₈O₃) + formic acid (CH₂O₂);
wherein HMF is 5-(hydroxymethyl)-2-furaldehyde, or 5-(hydroxymethyl)furfural. This mechanism of levulinic acid synthesis is described for example in the article by Meramo-Hurtado, Samir I. et al, ACS Omega 2019, 4, 27, 22302-22312.

However, the yield of this process is actually quite low, mainly due to the formation of numerous reaction by-products from which the levulinic acid must be separated through complex extraction and purification processes. In addition to various low molecular weight by-products, including formic acid, heat treatment of carbohydrates in aqueous solution at acidic pH leads to formation of humins, high molecular weight products resulting from condensation reactions. Humins typically separate as solids, usually dark in color, which cause numerous problems during the recovery process of levulinic acid.

WO 2012/162001 relates to a method for selectively dehydrating carbohydrates, (preferably sugars, e.g., fructose, glucose, xylose) to yield furanic compounds such as hydroxymethylfurfural and/or furfural, and methods to process these products further to yield derivatives such as furfuryl alcohol, levulinic acid, levulinic esters and/or γ-valerolactone.

US 2012/302767 relates to a method to make levulinic acid (LA), furfural, furfuryl alcohol, or gamma-valerolactone (GVL). The method comprises reacting in a first reactor a reactant comprising C₅ carbohydrates, C₆ carbohydrates, cellulose, or hemicellulose, or combinations thereof in: (i) a monophasic reaction medium comprising GVL and an acid; or (ii) a biphasic reaction system comprising an organic layer comprising GVL, and a substantially immiscible aqueous layer comprising water, an acid, and a sufficient concentration of a water-soluble solute to yield an aqueous solution that is substantially immiscible with the organic layer. At least a portion of C₅ carbohydrates, if present in the reactant, is converted into furfural, and at least a portion of C₆ carbohydrates, if present in the reactant, is converted to LA.

Patent application WO 98/19986 describes a process for producing levulinic acid, which comprises the treatment of a cellulose or hemicellulose-based biomass with a concentrated acid solution. The recovery of
levulinic acid is achieved by passing the reaction mixture through a chromatographic column, in particular a system of multiple chromatographic columns, known as "simulated moving bed chromatography": it is a very slow recovery process that requires the use of complex and expensive equipment.

In the state of the art, therefore, a strongly felt need remains to find an alternative process for the production of levulinic acid which has a reduced environmental impact and which guarantees satisfactory process yields on an industrial scale. In particular, one of the main objectives of the present invention is to produce levulinic acid by minimizing humins formation, which require complex and costly purification processes and involve a considerable reduction in levulinic acid yields. A further objective of the present invention is to carry out this process with the use of eco-compatible reagents and with raw materials from renewable sources.

The Applicant has now found that it is possible to achieve the above objectives and others which will be better illustrated below by means of a process for producing levulinic acid which comprises a catalytic conversion step of a pentose (in particular xylose or arabinose) into furfural in an organic solvent having a boiling temperature from 60°C to 220°C, followed by a step of reducing furfural to furfuryl alcohol, in the presence of a Lewis acid as catalyst and a protic solvent. Finally, furfuryl alcohol is converted into levulinic acid directly or indirectly, by preliminary conversion into a levulinic acid ester and its subsequent hydrolysis.

The present invention therefore relates to a process for producing levulinic acid, which comprises:
(a) converting a pentose into furfural, in the presence of an acid catalyst and an alkali or alkaline earth metal halide, in an organic solvent having a boiling temperature of 60°C to 220°C, at a temperature of 120°C at 200°C;
(b) reducing furfural to furfuryl alcohol, in a protic solvent and in the presence of a Lewis acid as catalyst;
(c) converting furfuryl alcohol into levulinic acid, directly or through conversion of furfuryl alcohol into an alkyl levulinate and subsequent hydrolysis of alkyl levulinate with formation of levulinic acid.

For the purposes of the present invention, in the description and claims that follow, the definitions of the numerical ranges include the individual values within the range and its extremes, unless otherwise specified.

For purposes of the present invention, in the description and claims that follow, the term "comprising" also includes the terms "which essentially consists of" or "which consists of".

Step (a) of the process according to the invention is carried out in the presence of an acid catalyst and an alkali or alkaline-earth metal halide. The acid catalyst can be of the homogeneous type, i.e. soluble in the reaction environment, or can be of the heterogeneous type, i.e. insoluble in the reaction environment.

In the first case, it is preferable to use an organic acid, in particular methanesulfonic acid CH₃-SO₂-OH (MSA). Alternatively, an inorganic Brønsted-Lowry acid (for example H₂SO₄, HCl) or a Lewis acid (for example AlCl₃, FeCl₃, ZrCl₄) can be used.

MSA is particularly advantageous, as it guarantees particularly high yields (around 80%). This acid is also advantageous from an environmental point of view, as it is a biodegradable product, halogen-, nitrogen- and phosphorus-free, and having high thermal stability and low corrosiveness. Furthermore, MSA can be easily recovered from the reaction environment by vacuum distillation.

In the case of a heterogeneous acid catalyst, this can consist in particular of an acid zeolite, preferably a βH zeolite.

Step (a) is carried out in an organic solvent having a boiling temperature from 60°C to 220°C, preferably from 100°C to 200°C. This solvent allows to operate at relatively high temperatures (from 120°C to 200°C) and this allows to significantly reduce the quantity of humins that can form due to secondary reactions involving the starting pentose when it is treated at high temperatures in an acidic environment.

Preferably, the organic solvent is selected from: γ-butyrolactone (GBL), γ-valerolactone (GVL), tetrahydrofuran (THF), dioxane, dimethylsulfoxide (DMS). More preferably, the organic solvent is γ-butyrolactone (GBL). It is a product with eco-compatibility characteristics, that is obtained through non-polluting processes and per se free from harmful effects on the environment.

Preferably, the organic solvent is at least partially miscible with water, so as to allow the reaction to be carried out in the presence of water. The presence of water minimizes the formation of humins and favors conversion to furfural. The organic solvent removes furfural from the aqueous environment, which, in the presence of the catalyst, could decompose, also forming humins. Preferably, the organic solvent is in admixture with water with a weight ratio of organic solvent to water from 50:50 to 98:2, more preferably from 80:20 to 95:5.

As for the halide, this is a halide of an alkali or alkaline earth metal. The halide is preferably chloride, bromide or iodide, more preferably iodide. The alkali metal is preferably selected from: lithium, sodium and potassium, while the alkaline earth metal is preferably selected from: magnesium, calcium, strontium, barium.

Without wishing to bind to an interpretative theory, it is believed that step (a) occurs according to the following mechanism (which is shown starting from xylose as initial pentose):

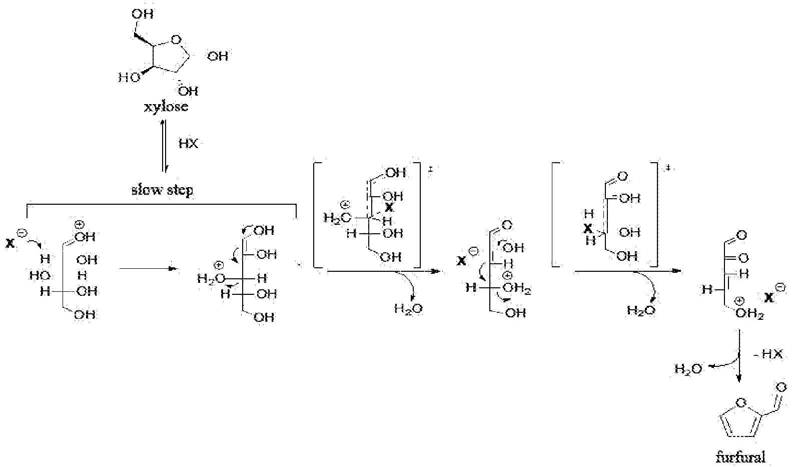

The halide substantially acts as a weak base via proton transfer in the first enolization step, which is the slow sub-step of step (a). During this sub-step the effectiveness of the halide is as follows: Cl>Br>I.

In the following three dehydration sub-steps, it is believed that the halide substantially has the function of stabilizing the two intermediate transition steps that are formed. In this case, the effectiveness of the halide is as follows: I>Br>Cl. Therefore, it may be convenient to use a mixture of two different halides, the first (for example a chloride) more effective in the first sub-step, the second (for example an iodide) more effective in the second sub-step. To avoid the use of a chloride, which poses disposal problems and is therefore not preferable from an environmental point of view, it is particularly convenient to use an iodide, which represents the best compromise of effectiveness for the different sub-steps of step (a).

Preferably, the acid catalyst is used in an amount of from 2% to 40% by weight, more preferably from 5% to 30% by weight, with respect to the weight of pentose.

Preferably, the halide is used in an amount of from 15% to 60% by weight, more preferably from 25% to 50% by weight, with respect to the weight of pentose.

Step (b) of the process is carried out in a protic solvent in the presence of a Lewis acid as catalyst. Preferably, step (b) is carried out in a heterogeneous step, i.e. the Lewis acid is substantially insoluble in the protic solvent.

Lewis acid is preferably selected from: zirconium oxide hydroxide (ZrO(OH)₂), zirconium hydroxide (Zr(OH)₄), zirconium oxide (ZrO₂), aluminum hydroxide (Al(OH)₃), titanium hydroxide (Ti(OH)₄), tin hydroxide (Sn(OH)₄), magnesium hydroxide (Mg(OH)₂). Preferably, the Lewis acid is in the form of nanoparticles.

Preferably, the Lewis acid is used in an amount of from 5% to 80% by weight, more preferably from 10% to 70% by weight, with respect to the weight of furfural.

Lewis acid can optionally be supported in order to facilitate its recovery, for example on an ion exchange resin, such as an Amberlyst resin (acid sulphonic resin) .

The protic solvent is preferably a C₁-C₆ alcohol, more preferably a C₃-C₆ secondary alcohol. Particularly preferred are isopropanol and isobutanol. Secondary alcohols act not only as solvents but also as reducing species (hydride donors), oxidized to the corresponding ketone, which is easily removed from the reaction environment.

Step (b) is preferably carried out at a temperature from 30°C to 150°C, more preferably from 50°C to 100°C.

Step (b) is believed to occur according to the Meerwein-Ponndorf-Verley (MPV) mechanism (see, for example, Boronat M. et al, J. Phys. Chem. B 2006, 110, 42, 21168-21174 - doi.org/10.1021/jp063249x).

This step (b) is particularly advantageous as it allows reduction of furfural to furfuryl alcohol to be achieved without using the common reducing agents based on hydrides and molecular hydrogen, ensuring a safe and sustainable process from an environmental point of view.

As for step (c), furfuryl alcohol can be directly converted into levulinic acid. Preferably, this conversion is carried out in the presence of an acid catalyst in an aqueous medium, in a homogeneous or heterogeneous phase.

In the case of homogeneous acid catalysis, it is preferable to use a Brønsted-Lowry acid in an aqueous medium, preferably selected from phosphoric acid (H₃PO₄) and methanesulfonic acid (MSA). MSA is particularly preferred, as it guarantees particularly high yields (around 80%).

Step (c) is preferably carried out at a temperature from 80°C to 160°C, preferably from 100°C to 150°C.

Preferably, the acid catalyst is used in an amount of from 30% to 120% by weight, more preferably from 70% to 100% by weight, with respect to the weight of furfuryl alcohol.

In the case of heterogeneous catalysis, a Lewis acid, for example AlCl₃, FeCl₃, ZrCl₄, or a Brønsted-Lowry acid, for example sulphonic silica (SiO₂-SO₃H), ion exchange resins, such as an Amberlyst resin (acid sulphonic resin), acid zeolites (e.g. βNH₄⁺ zeolite, βH zeolite, ZSM-5 zeolite), sulphonated activated carbons (AC-SO₃H).

As an alternative to direct conversion of furfuryl alcohol to levulinic acid in an aqueous medium, it is possible to obtain levulinic acid from furfuryl alcohol via a two-step process:
(c1) esterifying furfuryl alcohol with a C₁-C₄ alcohol to obtain an alkyl levulinate (levulinic acid alkyl ester);
(c2) hydrolyzing alkyl levulinate with the formation of levulinic acid.

Step (c1) is preferably carried out in the absence of water, so as to favor the formation of the ester and avoid secondary reactions that can lead to the formation of humins. Preferably, the C1-C4 alcohol is selected from: methanol, ethanol, propanol, butanol. Step (c2) is instead carried out in an aqueous environment, being a hydrolysis reaction.

Both steps (c1) and (c2) are preferably carried out in the presence of an acid catalyst (in homogeneous or heterogeneous phase), at a temperature from 80°C to 160°C, more preferably from 100°C to 150°C. The acid catalyst (the same or different for the two steps) can be selected from those indicated above for the direct conversion reaction of furfuryl alcohol to levulinic acid.

As regards the pentose used as initial substrate for the process of the present invention, this can be preferably selected from arabinose and xylose. These are products widely available in nature, constituting the basic monomer units of hemicellulose, a constituent of wood together with cellulose and lignin. Indicatively, in dry wood, cellulose represents 30-45%, lignin 20-30% and hemicellulose 10-25%.

It is also possible to obtain the pentose starting from a hexose or a mixture of hexoses. For example, the arabinose can be obtained from glucose, fructose and/or sucrose by removing a carbonaceous unit in the presence of βH zeolites, producing arabinose and formaldehyde as a by-product (see for example Jinglei Cui et al, Green Chem., 2016, 18, 1619-1624 and Luxin Zhang et al, Chem. Eng. J., 2017, 307, 868-876). The βH zeolite itself is able to dehydrate arabinose in situ to produce furfural, which leads to obtain levulinic acid in accordance with the process of the present invention.

Therefore, the process according to the present invention preferably comprises a preliminary step(a0), preceding step (a), in which a hexose selected from sucrose, fructose and glucose, or mixtures thereof, is subjected to removal of a carbonaceous unit in the presence of a zeolite (in particular a βH zeolite or a βFe zeolite) to obtain arabinose, which is converted in situ to furfural. The latter is then subjected to the subsequent steps (b) and (c).

Preferably, step (a0) is carried out in an organic solvent selected from those indicated above for step (a), optionally mixed with water. Particularly preferred is γ-butyrolactone (GBL). The reaction temperature is preferably between 140°C and 200°C, more preferably between 160°C and 190°C.

Step (a0) allows avoiding the formation of HMF (2,5-(hydroxymethyl)furfural) which is normally formed from hexoses and produces large quantities of humins, which, as already pointed out, constitute a significant problem from the operational point of view with considerable reductions in final yield.

The process according to the present invention can be summarized by the following general scheme, specifically referring to the two preferred pentoses, namely arabinose and xylose:

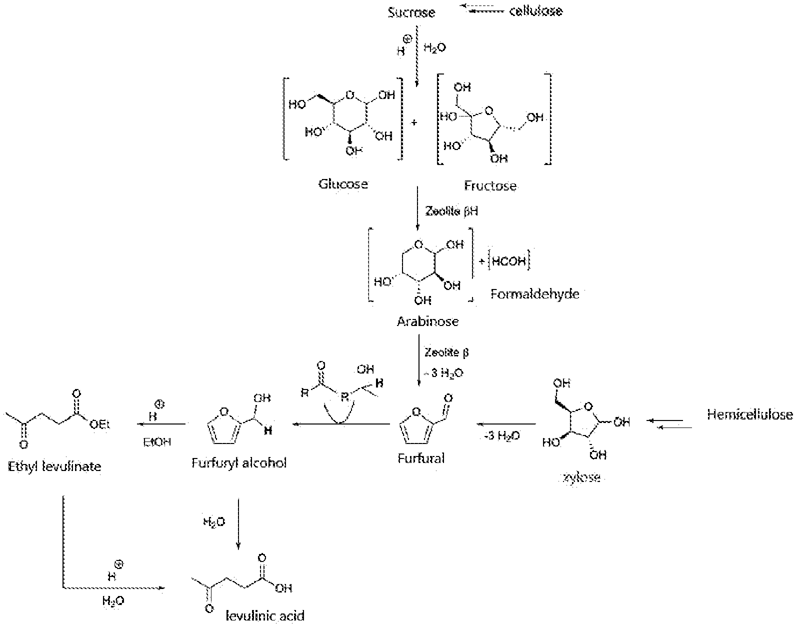

The following examples are intended for illustrative and not limitative purposes of the present invention.

### Example 1: Conversion of xylose to furfural.

### (a) Homogeneous catalysis.

A steel batch reactor equipped with magnetic stirrer was loaded with xylose (1.00 g), γ-butyrolactone (GBL, 16.8 mL), distilled water (1.0 mL; GBL/H₂O 95/5 w/w), potassium iodide (250 mg, 25% w/w) and methanesulfonic acid (200 mg, 20% w/w). Subsequently the reactor was sealed and the reaction mixture heated under stirring (200 rpm) at 150°C for 3 h. Once the reaction was complete, the reactor was cooled to room temperature and the reaction mixture filtered (porosity 0.45 µm) to obtain a clear solution. Molar yield (80%) and conversion (95%) were determined by analyzing a sample of the reaction mixture by HPLC method.

### (b) Heterogeneous catalysis.

A steel batch reactor equipped with magnetic stirrer was loaded with xylose (1.00 g), γ-butyrolactone (16.8 mL), distilled water (1.0 mL; GBL/H₂O 95/5 w/w), potassium iodide (500 mg, 50% w/w) and zeolite-βH (100 mg, 10% w/w). Subsequently the reactor was sealed and the reaction mixture heated under stirring (200 rpm) at 180°C for 2h. Once the reaction was complete, the reactor was cooled to room temperature and the reaction mixture centrifuged (5000 rpm) and filtered (porosity 0.45 µm) to obtain a clear solution. Molar yield (52%) and conversion (98%) were determined by analyzing a sample of the reaction mixture by HPLC method.

The scheme of the two reactions is as follows:
xylose -> furfural: Heterogeneous catalysis

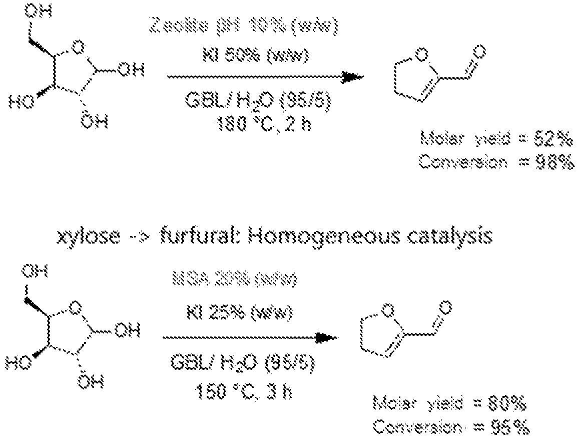

### Example 2: Reduction of furfural to furfuryl alcohol (heterogeneous catalysis).

A steel batch reactor equipped with a magnetic stirrer was loaded with furfural (1.00 g), isopropanol (20 mL) and zirconium oxide hydroxide (500 mg, 50% w/w). Subsequently the reactor was sealed and the reaction mixture heated under stirring (200 rpm) at 80°C for 24 h. Once the reaction was complete, the reactor was cooled to room temperature and the reaction mixture centrifuged (5000 rpm) and filtered (porosity 0.45 µm) to obtain a clear solution. Molar yield (100%) and conversion (100%) were determined by analyzing a sample of the reaction mixture by HPLC method.

The scheme of the reaction is as follows:
furfural -> furfuryl alcohol : Heterogeneous catalysis

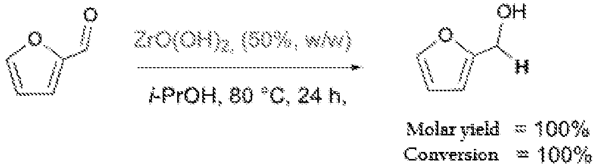

### Example 3: Direct conversion of furfuryl alcohol to levulinic acid.

### (a) Homogeneous catalysis.

A steel batch reactor equipped with magnetic stirrer was charged with furfuryl alcohol (1.00 g), THF (20 mL), distilled water (5.0 mL; THF/H₂O 4/1 v/v), and methanesulfonic acid (1.00 g, 100% w/w). Subsequently the reactor was sealed and the reaction mixture heated under stirring (200 rpm) at 140°C for 12 h. Once the reaction was complete, the reactor was cooled to room temperature and the reaction mixture filtered (porosity 0.45 µm) to obtain a clear solution. Molar yield (82%) and conversion (100%) were determined by analyzing a sample of the reaction mixture by HPLC method.

### (b) Heterogeneous catalysis.

A steel batch reactor equipped with magnetic stirrer was charged with furfuryl alcohol (1.00 g; 0.125 M), distilled water (81.6 mL) and Amberlyst 15 (24.5 g, 12 equiv. of a resin with 5 mmol/g loading). Subsequently the reactor was sealed and the reaction mixture heated under stirring (200 rpm) at 120°C for 1.5 h. Once the reaction was complete, the reactor was cooled to room temperature and the reaction mixture centrifuged (5000 rpm) and filtered (porosity 0.45 µm) to obtain a clear solution. Molar yield (48%) and conversion (100%) were determined by analyzing a sample of the reaction mixture by HPLC method.

The scheme of the two reactions is as follows:
furfuryl alcohol -> levulinic acid: Heterogeneous catalysis 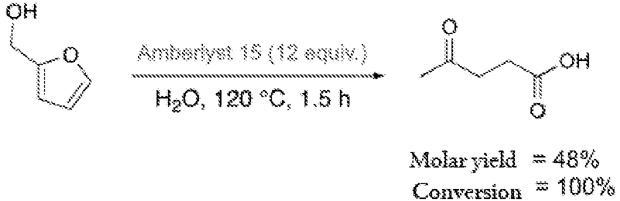
furfuryl alcohol - > levulinic acid: Homogeneous catalysis

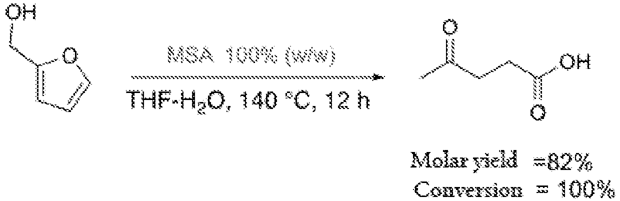

### Example 4: Conversion of furfuryl alcohol to ethyl levulinate.

### (a) Homogeneous catalysis.

A steel batch reactor equipped with a magnetic stirrer was charged with furfuryl alcohol (1.00 g), ethanol (30 mL) and methanesulfonic acid (1.00 g, 100% w/w). Subsequently the reactor was sealed and the reaction mixture heated under stirring (200 rpm) at 120°C for 12 h. Once the reaction was complete, the reactor was cooled to room temperature and the reaction mixture filtered (porosity 0.45 µm) to obtain a clear solution. Molar yield (95%) and conversion (100%) were determined by analyzing a sample of the reaction mixture by quantitative GC method.

### (b) Heterogeneous catalysis.

A steel batch reactor equipped with a magnetic stirrer was charged with furfuryl alcohol (1.00 g), ethanol (30 mL) and βNH₄⁺ zeolite (2.00 g, 200% w/w). Subsequently the reactor was sealed and the reaction mixture heated under stirring (200 rpm) at 120°C for 8 h. Once the reaction was complete, the reactor was cooled to room temperature and the reaction mixture centrifuged (5000 rpm) and filtered (porosity 0.45 µm) to obtain a clear solution. Molar yield (78%) and conversion (100%) were determined by analyzing a sample of the reaction mixture by quantitative GC method.

The scheme of the two reactions is as follows:
furfuryl alcohol -> Ethyl levulinate: Heterogeneous catalysis 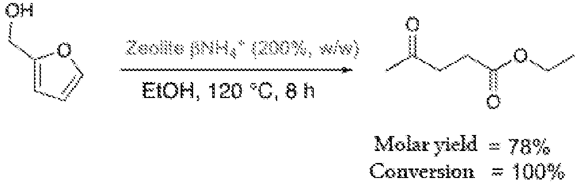
furfuryl alcohol -> Ethyl levulinate: Homogeneous catalysis

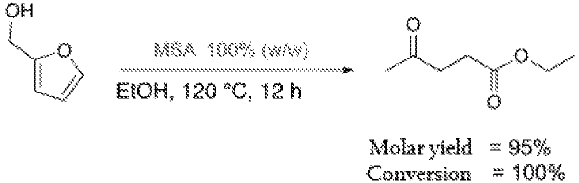

### Example 5: Hydrolysis of ethyl levulinate to obtain levulinic acid.

### (a) Homogeneous catalysis.

A steel batch reactor equipped with a magnetic stirrer was charged with ethyl levulinate (1.00 g), distilled water (30 mL) and methanesulfonic acid (153 mg, 15.3% w/w). Subsequently the reactor was sealed and the reaction mixture heated under stirring (200 rpm) at 120°C for 18 h. Once the reaction was complete, the reactor was cooled to room temperature and the reaction mixture filtered (porosity 0.45 µm) to obtain a clear solution. Molar yield (95%) and conversion (95%) were determined by analyzing a sample of the reaction mixture by HPLC method.

### (b) Heterogeneous catalysis.

A steel batch reactor equipped with a magnetic stirrer was loaded with ethyl levulinate (1.00 g), distilled water (30 mL) and βH zeolite (153 mg, 15.3% w/w). Subsequently the reactor was sealed and the reaction mixture heated under stirring (200 rpm) at 120°C for 18 h. Once the reaction was complete, the reactor was cooled to room temperature and the reaction mixture centrifuged (5000 rpm) and filtered (porosity 0.45 µm) to obtain a clear solution. Molar yield (90%) and conversion (95%) were determined by analyzing a sample of the reaction mixture by HPLC method.

The scheme of the two reactions is as follows:
**Ethyl levulinate -> Levulinic acid: Heterogeneous catalysis** 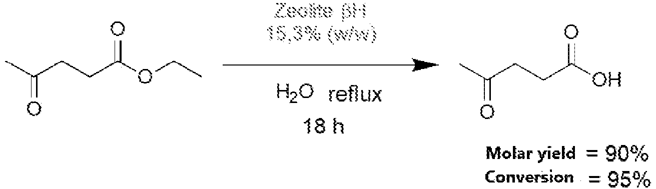
**Ethyl levulinate -> Levulinic acid: Homoogeneous catalysis**

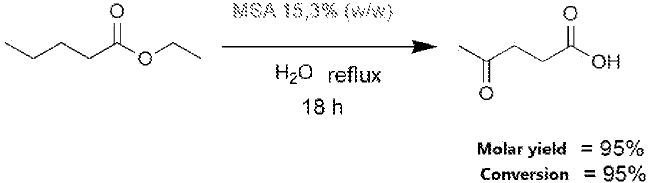

### Example 6: Conversion of sucrose into furfural through arabinose formation (heterogeneous catalysis).

A steel batch reactor equipped with magnetic stirrer was loaded with sucrose (1.00 g), γ-butyrolactone (16.8 mL), distilled water (1.0 mL; GBL/ H₂O 95/5 w/w), potassium iodide (500 mg, 50% w/w) and βH-zeolite (100 mg, 10% w/w). Subsequently the reactor was sealed and the reaction mixture heated under stirring (200 rpm) at 180°C for 2 h. Once the reaction was complete, the reactor was cooled to room temperature and the reaction mixture centrifuged (5000 rpm) and filtered (porosity 0.45 µm) to obtain a clear solution. Molar yield (65%) and conversion (88%) were determined by analyzing a sample of the reaction mixture by HPLC method.

The scheme of the reaction is as follows:
**Sucrose -> Furfural: Heterogeneous catalysis**

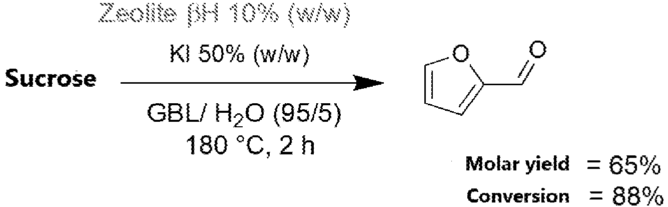

## Claims

1. Process for producing levulinic acid, which comprises:
(a) converting a pentose into furfural, in the presence of an acid catalyst and of an alkali or alkaline earth metal halide, in an organic solvent having a boiling temperature from 60°C to 220°C, at a temperature from 120°C to 200°C;
(b) reducing furfural to furfuryl alcohol, in a protic solvent and in the presence of a Lewis acid as catalyst;
(c) converting furfuryl alcohol into levulinic acid, either directly or by converting furfuryl alcohol into an alkyl levulinate and subsequent hydrolysis of alkyl levulinate with formation of levulinic acid;
wherein in step (a) the pentose is selected from arabinose and xylose.

2. Process according to claim 1, wherein the acid catalyst of step (a) is an organic acid, preferably methanesulfonic acid CH₃-SO₂-OH (MSA) .

3. Process according to claim 1, wherein the acid catalyst of step (a) is a Brønsted-Lowry inorganic acid or a Lewis acid.

4. Process according to claim 1, wherein the acid catalyst of step (a) is an acid zeolite, preferably a βH zeolite.

5. Process according to any one of the preceding claims, wherein in step (a) the organic solvent is selected from: γ-butyrolactone (GBL), γ-valerolactone (GVL), tetrahydrofuran (THF), dioxane, dimethylsulfoxide (DMS), possibly in admixture with water.

6. Process according to any one of the preceding claims, wherein in step (a) the halide is an iodide.

7. Process according to any one of the preceding claims, wherein step (b) is carried out in heterogeneous phase, i.e. the Lewis acid is substantially insoluble in the protic solvent.

8. Process according to claim 7, wherein in step (b) the Lewis acid is selected from: zirconium oxide hydroxide (ZrO(OH)₂), zirconium hydroxide (Zr(OH)₄), zirconium oxide (ZrO₂), aluminum hydroxide (Al(OH)₃), titanium hydroxide (Ti(OH)₄), tin hydroxide (Sn(OH)₄), magnesium hydroxide (Mg(OH)₂).

9. Process according to any one of the preceding claims, wherein the protic solvent of step (b) is a C₁-C₆ alcohol, preferably a C₃-C₆ secondary alcohol.

10. Process according to any one of the preceding claims, wherein step (b) is carried out at a temperature from 30°C to 150°C, preferably from 50°C to 100°C.

11. Process according to any one of the preceding claims, wherein in step (c) the furfuryl alcohol is directly converted into levulinic acid in the presence of an acid catalyst in an aqueous medium, in a homogeneous or heterogeneous phase.

12. Process according to claim 11, wherein the acid catalyst is a Brønsted-Lowry acid, preferably selected from phosphoric acid (H₃PO₄) and methanesulfonic acid (MSA).

13. Process according to claim 11, wherein the acid catalyst is a Lewis acid, for example AlCl₃, FeCl₃, ZrCl₄, or a Brønsted-Lowry acid, for example sulphonic silica (SiO₂-SO₃H), ion exchange resins, acid zeolites, sulphonated activated carbons (AC-SO₃H).

14. Process according to any one of the preceding claims, wherein step (c) is carried out at a temperature from 80°C to 160°C, preferably from 100°C to 150°C.

15. Process according to any one of claims 1 to 10, wherein in step (c) furfuryl alcohol is converted to levulinic acid in two steps:
(c1) esterifying furfuryl alcohol with a C₁-C₄ alcohol to obtain an alkyl levulinate (levulinic acid alkyl ester);
(c2) hydrolyzing the alkyl levulinate with formation of levulinic acid.

16. Process according to claim 15, wherein step (c1) is carried out in the absence of water.

17. Process according to claim 16, wherein step (c2) is carried out in an aqueous environment.

18. Process according to any one of claims 15 to 17, wherein both steps (c1) and (c2) are carried out in the presence of an acid catalyst, at a temperature from 80°C to 160°C, preferably from 100°C to 150°C.

19. Process according to any one of the preceding claims, which comprises a preliminary step (a0), preceding step (a), wherein a hexose selected from sucrose, fructose and glucose, or mixtures thereof, is subjected to removal of a carbonaceous unit in the presence of a zeolite to obtain arabinose, which is converted in situ to furfural.

## Patentansprüche

1. Verfahren zur Herstellung von Levulinsäure, das Folgendes umfasst:
(a) Umwandlung einer Pentose in Furfural in Gegenwart eines sauren Katalysators und eines Alkali- oder Erdalkalimetallhalogenids in einem organischen Lösungsmittel mit einer Siedetemperatur von 60°C bis 220°C bei einer Temperatur von 120°C bis 200°C;
(b) Reduktion von Furfural zu Furfurylalkohol in einem protischen Lösungsmittel und in Gegenwart einer Lewis-Säure als Katalysator;
(c) Umwandlung von Furfurylalkohol in Levulinsäure, entweder direkt oder durch Umwandlung von Furfurylalkohol in ein Alkylevulinat und anschließende Hydrolyse von Alkylevulinat unter Bildung von Levulinsäure;
wobei in Schritt (a) die Pentose aus Arabinose und Xylose ausgewählt wird.

2. Verfahren nach Anspruch 1, wobei der saure Katalysator von Schritt (a) eine organische Säure ist, vorzugsweise Methansulfonsäure CH₃-SO₂-OH (MSA).

3. Verfahren nach Anspruch 1, wobei der Säurekatalysator von Schritt (a) eine anorganische Brønsted-Lowry-Säure oder eine Lewis-Säure ist.

4. Verfahren nach Anspruch 1, wobei der Säurekatalysator von Schritt (a) ein saurer Zeolith, vorzugsweise ein βH-Zeolith, ist.

5. Verfahren nach einem der vorhergehenden Ansprüche, wobei in Schritt (a) das organische Lösungsmittel ausgewählt wird aus: γ-Butyrolacton (GBL), γ-Valerolacton (GVL), Tetrahydrofuran (THF), Dioxan, Dimethylsulfoxid (DMS), gegebenenfalls im Gemisch mit Wasser.

6. Verfahren nach einem der vorhergehenden Ansprüche, wobei in Schritt (a) das Halogenid ein Iodid ist.

7. Verfahren nach einem der vorhergehenden Ansprüche, wobei Schritt (b) in heterogener Phase durchgeführt wird, d.h. die Lewis-Säure ist in dem protischen Lösungsmittel im Wesentlichen unlöslich.

8. Verfahren nach Anspruch 7, wobei in Schritt (b) die Lewis-Säure ausgewählt wird aus:
Zirkoniumoxidhydroxid (ZrO(OH)₂), Zirkoniumhydroxid (Zr(OH)₄), Zirkoniumoxid (ZrO₂),
Aluminiumhydroxid (Al(OH)₃), Titanhydroxid (Ti(OH)₄), Zinnhydroxid (Sn(OH)₄), Magnesiumhydroxid (Mg(OH)₂).

9. Verfahren nach einem der vorhergehenden Ansprüche, wobei das protische Lösungsmittel von Schritt (b) ein C₁-C₆ Alkohol, vorzugsweise ein C₃-C₆ sekundärer Alkohol ist.

10. Verfahren nach einem der vorhergehenden Ansprüche, wobei Schritt (b) bei einer Temperatur von 30°C bis 150°C, vorzugsweise von 50°C bis 100°C, durchgeführt wird.

11. Verfahren nach einem der vorhergehenden Ansprüche, wobei in Schritt (c) der Furfurylalkohol in Gegenwart eines sauren Katalysators in einem wässrigen Medium in einer homogenen oder heterogenen Phase direkt in Levulinsäure umgewandelt wird.

12. Verfahren nach Anspruch 11, wobei der saure Katalysator eine Brønsted-Lowry-Säure ist, vorzugsweise ausgewählt aus Phosphorsäure (H₃PO₄) und Methansulfonsäure (MSA).

13. Verfahren nach Anspruch 11, wobei der saure Katalysator eine Lewis-Säure, zum Beispiel AlCl₃, FeCl₃, ZrCl₄, oder eine Brønsted-Lowry-Säure ist, zum Beispiel Sulfonsäure (SiO₂-SO₃H), Ionenaustauscherharze, saure Zeolithe, sulfonierte Aktivkohlen (AC-SO₃H).

14. Verfahren nach einem der vorhergehenden Ansprüche, wobei Schritt (c) bei einer Temperatur von 80°C bis 160°C, vorzugsweise von 100°C bis 150°C, durchgeführt wird.

15. Verfahren nach einem der Ansprüche 1 bis 10, wobei in Schritt (c) Furfurylalkohol in zwei Schritten in Levulinsäure umgewandelt wird:
(c1) Veresterung von Furfurylalkohol mit einem C₁-C₄ Alkohol, um ein Alkylevulinat (Levulinsäurealkylester) zu erhalten;
(c2) Hydrolyse des Alkylevulinats unter Bildung von Levulinsäure.

16. Verfahren nach Anspruch 15, wobei der Schritt (c1) in Abwesenheit von Wasser durchgeführt wird.

17. Verfahren nach Anspruch 16, wobei Schritt (c2) in einer wässrigen Umgebung durchgeführt wird.

18. Verfahren nach einem der Ansprüche 15 bis 17, wobei beide Schritte (c1) und (c2) in Gegenwart eines Säurekatalysators bei einer Temperatur von 80°C bis 160°C, vorzugsweise von 100°C bis 150°C, durchgeführt werden.

19. Verfahren nach einem der vorangehenden Ansprüche, das einen vorbereitenden Schritt (a0) umfasst, der dem Schritt (a) vorausgeht, in dem eine Hexose, ausgewählt aus Saccharose, Fructose und Glucose oder deren Mischungen, in Gegenwart eines Zeolithen der Entfernung einer kohlenstoffhaltigen Einheit unterzogen wird, um Arabinose zu erhalten, die in situ in Furfural umgewandelt wird.

## Revendications

1. Procédé de production d'acide lévulinique comprenant:
(a) conversion d'un pentose en furfural, en présence d'un catalyseur acide et d'un halogénure de métal alcalin ou alcalino-terreux, dans un solvant organique dont la température d'ébullition est comprise entre 60°C et 220°C, à une température comprise entre 120°C et 200°C;
(b) réduction du furfural en alcool furfurylique, dans un solvant protique et en présence d'un acide de Lewis comme catalyseur;
(c) conversion de l'alcool furfurylique en acide lévulinique, soit directement, soit par conversion de l'alcool furfurylique en lévulinate d'alkyle et hydrolyse ultérieure du lévulinate d'alkyle avec formation d'acide lévulinique;
dans lequel, à l'étape (a), le pentose est choisi parmi l'arabinose et le xylose.

2. Procédé selon la revendication 1, dans lequel le catalyseur acide de l'étape (a) est un acide organique, de préférence l'acide méthanesulfonique CH₃-SO₂-OH (MSA).

3. Procédé selon la revendication 1, dans lequel le catalyseur acide de l'étape (a) est un acide inorganique de Brønsted-Lowry ou un acide de Lewis.

4. Procédé selon la revendication 1, dans lequel le catalyseur acide de l'étape (a) est une zéolithe acide, de préférence une zéolithe βH.

5. Procédé selon l'une quelconque des revendications précédentes, dans lequel à l'étape (a) le solvant organique est choisi parmi: γ-butyrolactone (GBL), γ-valérolactone (GVL), tétrahydrofurane (THF), dioxane, diméthylsulfoxyde (DMS), éventuellement en mélange avec de l'eau.

6. Procédé selon l'une quelconque des revendications précédentes, dans lequel, à l'étape (a), l'halogénure est un iodure.

7. Procédé selon l'une quelconque des revendications précédentes, dans lequel l'étape (b) est réalisée en phase hétérogène, c'est-à-dire que l'acide de Lewis est sensiblement insoluble dans le solvant protique.

8. Procédé selon la revendication 7, dans lequel à l'étape (b) l'acide de Lewis est choisi parmi: l'hydroxyde d'oxyde de zirconium (ZrO(OH)₂), l'hydroxyde de zirconium (Zr(OH)₄), l'oxyde de zirconium (ZrO₂), hydroxyde d'aluminium (Al(OH)₃), hydroxyde de titane (Ti(OH)₄), hydroxyde d'étain (Sn(OH)₄), hydroxyde de magnésium (Mg(OH)₂).

9. Procédé selon l'une quelconque des revendications précédentes, dans lequel le solvant protique de l'étape (b) est un alcool C₁-C₆, de préférence un alcool secondaire C₃-C₆.

10. Procédé selon l'une quelconque des revendications précédentes, dans lequel l'étape (b) est effectuée à une température comprise entre 30°C et 150°C, de préférence entre 50°C et 100°C.

11. Procédé selon l'une quelconque des revendications précédentes, dans lequel à l'étape (c) l'alcool furfurylique est directement converti en acide lévulinique en présence d'un catalyseur acide en milieu aqueux, en phase homogène ou hétérogène.

12. Procédé selon la revendication 11, dans lequel le catalyseur acide est un acide de Brønsted-Lowry, de préférence choisi parmi l'acide phosphorique (H₃PO₄) et l'acide méthanesulfonique (MSA).

13. Procédé selon la revendication 11, dans lequel le catalyseur acide est un acide de Lewis, par exemple AlCl₃, FeCl₃, ZrCl₄, ou un acide de Brønsted-Lowry, par exemple la silice sulfonique (SiO₂-SO₃H), les résines échangeuses d'ions, les zéolithes acides, les charbons actifs sulfonés (AC-SO₃H).

14. Procédé selon l'une quelconque des revendications précédentes, dans lequel l'étape (c) est effectuée à une température comprise entre 80°C et 160°C, de préférence entre 100°C et 150°C.

15. Procédé selon l'une quelconque des revendications 1 à 10, dans lequel, à l'étape (c), l'alcool furfurylique est converti en acide lévulinique en deux étapes:
(c1) estérifier l'alcool furfurylique avec un alcool C₁-C₄ pour obtenir un lévulinate d'alkyle (ester d'alkyle de l'acide lévulinique);
(c2) hydrolyse du lévulinate d'alkyle avec formation d'acide lévulinique.

16. Procédé selon la revendication 15, dans lequel l'étape (c1) est effectuée en l'absence d'eau.

17. Procédé selon la revendication 16, dans lequel l'étape (c2) est réalisée en milieu aqueux.

18. Procédé selon l'une quelconque des revendications 15 à 17, dans lequel les deux étapes (c1) et (c2) sont effectuées en présence d'un catalyseur acide, à une température de 80°C à 160°C, de préférence de 100°C à 150°C.

19. Procédé selon l'une quelconque des revendications précédentes, qui comprend une étape préliminaire (a0), précédant l'étape (a), dans laquelle un hexose choisi parmi le saccharose, le fructose et le glucose, ou leurs mélanges, est soumis à l'élimination d'une unité carbonée en présence d'une zéolithe pour obtenir de l'arabinose, qui est converti in situ en furfural.
